# EUROPEAN PATENT APPLICATION

(11) **EP 4 837 585 A1**
(43) Date of publication of application: **11.11.2026**
(21) Application number: 26177703.1
(22) Date of filing: 08.05.2026
(51) Int. Cl.: A61K 36/48, A23L 33/00, A61K 31/352, A61K 31/353, A61K 31/7034, A61K 33/04, A61K 33/30, A61K 36/53, A61K 36/82, A61P 13/08

(54) **MIXTURE COMPRISING EXTRACTS OF CAMELLIA SINENSIS (L.) KUNTZE, TRIFOLIUM PRATENSE L. AND AJUGA REPTANS L., COMPOSITION COMPRISING SUCH MIXTURE AND THEIR USE IN THE TREATMENT OF PROSTATE INFLAMMATION AND/OR BENIGN PROSTATIC HYPERPLASIA**

(30) Priority: 08.05.2025 IT 202500010395
(71) Applicant: Global Pharmacies Partner S.R.L. in Forma Abbreviata G.P.P. S.R.L., 20122 Milano (IT)
(72) Inventor: TORELLO, Giulio, 20123 Milano (IT); CALI', Riccardo, 20123 Milano (IT)
(74) Representative: Marben S.r.l.

(57) **Abstract**

The present invention relates to a mixture, pharmaceutical and/or nutraceutical compositions comprising that mixture and the use thereof in therapy, preferably in a method for reducing and/or treating prostate inflammation, and/or in a method for reducing and/or treating benign prostatic hyperplasia.

Said mixture comprises or, alternatively, consists of:
i) an epigallocatechin-3-gallate (EGCG) and/or an extract of *Camellia sinensis (L.) Kuntze;*
ii) one or more isoflavones selected in the group comprising or, alternatively, consisting of formononetin, daidzein, genistein, biochanin A and/or an extract of red clover *Trifolium pratense L. Leguminosa;* and
iii) a phenylpropanoid teupolioside and/or an extract of *Ajuga Reptans L.*

## Description

The present invention relates to a mixture, pharmaceutical and/or nutraceutical compositions comprising that mixture and the use thereof in therapy, preferably in a method for reducing and/or treating prostate inflammation, and/or in a method for reducing and/or treating benign prostatic hyperplasia.

Said mixture comprises or, alternatively, consists of:
i) an epigallocatechin-3-gallate (EGCG) and/or an extract of *Camellia sinensis (L.) Kuntze*;
ii) one or more isoflavones selected in the group comprising or, alternatively, consisting of formononetin, daidzein, genistein, biochanin A and/or an extract of red clover *Trifolium pratense L. Leguminosa*; and
iii) a phenylpropanoid teupolioside and/or an extract of *Ajuga Reptans L.*

### BACKGROUND OF THE INVENTION

The pathologies or symptoms correlated with the prostate are a public health issue with a considerable impact from an epidemiological, clinical and economic viewpoint.

The prostate is a glandular organ of the male genital apparatus whose function is to produce and emit prostatic fluid, one of the constituents of sperm, which contains the elements necessary to nourish and convey spermatozoa.

In particular, prostate inflammation is a very widespread problem which, according to statistics, affects 8.2% of men on at least one occasion in their lifetime.

Prostate inflammation, known in the medical field by the term "prostatitis", may depend on infective causes (bacterial, to be precise) or non-infective ones.

Medical pathology recognizes four types of prostatitis:
- acute prostatitis of bacterial origin (or also acute bacterial prostatitis or prostatitis type I);
- chronic prostatitis of bacterial origin (or also chronic bacterial prostatitis or prostatitis type II);
- chronic prostatitis of non-bacterial origin (or also chronic non-bacterial prostatitis or prostatitis type III); and
- asymptomatic prostatitis (or prostatitis type IV).

Acute bacterial prostatitis or prostatitis type I is caused by infection on the part of bacteria (*Escherichia coli, Klebsiella*, *Proteus*, *Pseudomonas*, *Enterobacteria*, *Enterococcus*, *Serratia* and *Staphylococcus aureus* being among the most common). Subjects affected by acute prostatitis report shivers, fever, and pain in the back and in the genital area, burning and discomfort during the urination, and widespread aching. Acute urine retention is also common due to the fact that the inflammation causes a narrowing of the first portion of the urethra (prostatic urethra). Antibiotics represent the first-line treatment in acute prostatitis.

Chronic bacterial prostatitis or prostatitis type II is a chronic prostate inflammation which may derive from the lack of treatment of an acute prostatitis of bacterial origin, a urinary tract infection or a testicular infection (e.g. epididymitis).

The therapy requires lengthy cycles of antibiotics with high penetrability of the prostate gland. However, it has been observed that β-lactams and nitrofurantoin are ineffective and, in the case of persistent infections, 80% of patients, but not everyone, may benefit from an improvement in symptoms.

Prostatitis is an inflammation of the prostate. It would be appropriate to call it urethral prostatitis, since the first portion of the urethra, passing through the prostate, is affected by the inflammation.

Chronic abacterial prostatitis or chronic pelvic pain syndrome (CPPS) can be of an inflammatory type (category IIIa) or non-inflammatory type (category IIIb). In the first type, the urine, sperm or seminal fluid contain pus (dead leukocytes), whereas in the second type no pus or leukocyte residues are present. Recent studies have called into question the distinction between the two categories, considering that in both cases there is evidence of inflammation, considering more complex inflammatory markers such as cytokines.

The therapy consists in a combination of a psychological therapy, physiotherapy, stretching exercises which help to obtain greater relaxation of the pelvic floor, and pharmacological therapy based on alpha-blockers, antibiotics, anti-inflammatories, 5-alpha reductase inhibitors and drugs for neuropathic pain. This pharmacological therapy commits the patient for at least 3 months.

In view of this, it is thus evident that the high involvement of this therapy in the patient's life causes, as an indirect consequence, an increase in psychological stress that in no way favors - indeed it hinders - the cure of the subject affected by prostatitis.

In fact, the theory whereby, in some predisposed subjects, stress may lead to muscular tension in the pelvic area which increases with a neurological feedback mechanism (neural wind-up), is well known.

Finally, asymptomatic prostatitis or prostatitis type IV is prostatitis which, in the affected men, does not produce any symptom, though it is responsible for a marked inflammatory process at the prostate level. The therapy is based on the administration of antibiotics and/or anti-inflammatories.

Another very common pathology that affects the prostate and is accompanied by inflammation is benign prostatic hyperplasia (BPH).

Benign prostatic hyperplasia is one of the most widespread diseases which occurs in men with aging, especially after 40-50 years, and it is characterized by an increase in volume of the prostate gland due to the proliferation of epithelial and stromal prostate cells and the creation of nodules which, as the years pass, increase in number and size and compress and distort the prostatic urethra, producing an obstruction that hinders urine outflow.

Changes caused by hormones, in particular testosterone and dihydrotestosterone (DHT), seem to be the most frequent causes. It is not infrequent for situations of prostatic hyperplasia to be associated with prostatic inflammation.

In these cases, the inflammation, together with the prostatic hyperplasia, brings about a loss of elasticity in the prostate, urinary blockage and, in the most serious cases, overflow incontinence.

From a molecular viewpoint, in cases of prostatic hypertrophy one observes an overexpression of 5-alpha reductase (5αR), an increase in dihydrotestosterone levels with a consequent increase in PSA (prostate-specific antigen) and an increase in the proliferation of stromal and epithelial cells, and the release of inflammation mediators and free radicals. Lastly, the increase in the proliferation of stromal and epithelial cells may cause the compression of the urethral canal and the appearance of LUTS *(*lower urinary tract symptoms*).*

It should be noted that the prostate-specific antigen (PSA) is not a diagnostic parameter of prostatitis. In fact, if this value is measured by means of the PSA test in a phase of acute infection, it can show to be very high, but it does not have clinical significance. In order to have clinical significance, it must be measured three months after the resolution of symptoms. As said, PSA is not a diagnostic parameter of prostatitis, but it can be very high in the acute phase because of the inflammation. Values ranging between 4 and 10 ng/ml indicate that the prostate has a problem which could be of infective origin or benign inflammatory (prostatitis) origin (benign prostatic hypertrophy, BPH). However, the PSA in the blood does not allow distinguishing with certainty a prostate disease of the benign type from prostate cancer.

A very high PSA value can derive from an inflammatory and/or neoplastic component.

It would be desirable to be able to have a non-pharmacological therapeutic solution which, in the case of a subject with a high PSA value, is capable of reducing the PSA value linked solely to the inflammatory component and not to the neoplastic one. This is because if the therapeutic solution acted by strongly reducing the high PSA value and, therefore, acted on both the inflammatory component and the neoplastic one, said therapeutic solution, not having an effectiveness on the neoplasia because it is not a drug, would convey an incorrect message to said subject, making the subject believe that the problem has been overcome whereas in actual fact it has not, because he has a neoplasia and not an inflammation.

This false message, by not reflecting the real situation, where the neoplasia persists, only shifts the clinical diagnosis with the risk of aggravating the situation.

In other words, it would be desirable to have a non-pharmacological therapeutic solution which physiologically reduces the elevated PSA value given only by the component linked to the inflammation, but not that linked to the neoplasia.

WO 2017/108907 discloses a composition comprising an extract of *Ajuga reptans* containing from 10% to 50% teupolioside relative to the total weight of the phenylpropanoids in the extract, and an *Epilobium* extract.

The pharmacological treatment of benign prostatic hyperplasia is based on the use of alpha-blockers (α1-adrenergic receptor antagonists) such as doxazosin, terazosin, alfuzosin, silodosin and tamsulosin. However, alpha-blockers may cause retrograde ejaculation.

5α-reductase inhibitors like finasteride and dutasteride are also used.

In the case of failure of the medical treatment, it is necessary to perform a surgical and, precisely, invasive treatment of resection of part of the prostate through the urethra (transurethral resection of the prostate, TURP).

In order to avoid invasive surgical treatments, it is thus necessary to reduce the prostate inflammation and provide a mixture or composition useful for treating benign prostatic hyperplasia and the inflammation associated therewith, which does not cause side effects. These objects and still others, which will become clear from the detailed description that follows, are achieved by a mixture comprising or, alternatively, consisting of i) an epigallocatechin-3-gallate (EGCG) and/or an extract of *Camellia sinensis (L.) Kuntze*; ii) one or more isoflavones selected in the group comprising or, alternatively, consisting of formononetin, daidzein, genistein, biochanin A and/or an extract of red clover *Trifolium pratense L. Leguminosa*; and iii) a phenylpropanoid teupolioside and/or an extract of *Ajuga Reptans* L. and the composition comprising that mixture (mixture and/or composition of the invention), which have the technical features claimed in the appended claims.

### SUMMARY OF THE INVENTION

The technical problem that the present invention addresses and resolves is to provide a mixture of natural active ingredients, and nutraceutical and/or pharmaceutical compositions comprising them, capable of reducing and/or treating prostate inflammation, and particularly useful for treating benign prostatic hyperplasia.

Inflammation (or phlogosis) is a fundamental defense reaction the body initiates when it undergoes tissue damage.

This response is mainly prompted by three factors: infections (caused by microbes or their toxins), direct cell damage which releases alarm signals, and alterations in the body's internal balance (homeostasis). This latter aspect is crucial in the prostate, where hormonal imbalances between testosterone and DHT or between androgens and estrogens may act as a veritable trigger for chronic inflammation, alongside the classic bacterial or viral causes.

When the body is exposed at length to harmful stimuli or internal imbalances, the inflammation becomes chronic. This condition is characterized by a continuous accumulation of lymphocytes, which, over time, may progressively damage the affected tissues. Today we know with certainty that this mechanism plays a key role in the development of lower urinary tract symptoms and disorders (LUTS). Chronic prostate inflammation aggravates both benign prostatic hypertrophy (BPH) and chronic pelvic pain syndrome, confirming the close clinical link existing between these conditions.

Furthermore, the present invention provides a non-pharmacological therapeutic solution capable of reducing an elevated PSA value to physiological values by acting on the inflammatory component responsible for such an elevated PSA value and, therefore, useful in that it can be administered to subjects who for the first time find themselves with an elevated PSA value.

Following an intense research activity, the Applicant has developed and fine-tuned a mixture comprising or, alternatively, consisting of specific active components capable of reducing prostate inflammation and, in the meantime, of acting on the inhibition of prostate cell proliferation, which is typical of benign prostatic hyperplasia.

A first aspect of the present invention relates to a mixture comprising or, alternatively, consisting of:
i) an epigallocatechin-3-gallate (EGCG) and/or an extract of *Camellia sinensis (L.) Kuntze*;
ii) one or more isoflavones selected in the group comprising or, alternatively, consisting of formononetin, daidzein, genistein, biochanin A and/or an extract of red clover *Trifolium pratense L. Leguminosa*; and
iii) a phenylpropanoid teupolioside and/or an extract of *Ajuga Reptans L.*

Preferably, in combination or association with components i) + ii) + iii), the mixture may further comprise:
iv) selenium or a salt thereof and/or selenium in an organic form, preferably selected in the group comprising or, alternatively, consisting of L-selenomethionine, sodium selenite, sodium selenate, selenocysteine and/or mixtures thereof, more preferably L-selenomethionine; and/or
v) zinc or a salt thereof and/or zinc in an organic form, preferably selected in the group comprising or, alternatively, consisting of zinc gluconate, zinc picolinate, zinc acetate, zinc bisglycinate, zinc sulfate and zinc citrate and/or mixtures thereof, more preferably zinc citrate and/or mixtures thereof.

Therefore, more preferably, the mixture comprises or, alternatively, consists of:
i) an epigallocatechin-3-gallate (EGCG) and/or a dry extract of *Camellia sinensis (L.) Kuntze*;
ii) one or more isoflavones selected in the group comprising or, alternatively, consisting of formononetin, daidzein, genistein, biochanin A and/or a dry extract of red clover *Trifolium pratense L. Leguminosa*;
iii) a phenylpropanoid teupolioside and/or a dry extract of *Ajuga Reptans L*;
iv) selenium or a salt thereof; and/or
v) zinc or a salt thereof.

In the context of the present invention, the term "mixture" indicates the set of active components that exert a beneficial effect, preferably a biological effect.

In addition to the above-described components, if desired or necessary, the mixture of the invention can comprise further active components.

A second aspect of the present invention relates to a pharmaceutical and/or nutraceutical composition comprising the mixture according to the first aspect of the invention and food or pharmaceutical grade excipients and/or additives.

In the context of the present invention, the term "composition" is meant to indicate the set of active components present in the mixture and the additives and/or excipients and/or further components which are not active components. Even if not expressly indicated, the term "composition" in the present description includes pharmaceutical compositions, nutraceutical compositions, food compositions or compositions for medical devices pursuant to Regulation (EU) 2017/745 (briefly, the composition(s)of the present invention) or compositions for supplements and foods for special medical purposes (FSMPs). In the context of the present invention, -selenium- is understood to include a salt or an organic form of selenium such as, for example, L-selenomethionine in the various hydrated forms thereof, capable of rendering selenium bioavailable and absorbable, preferably in the form of a cation or elementary selenium, by the body once administered thereto.

In the context of the present invention, -zinc- is understood to include a salt or an organic form of zinc such as, for example, zinc citrate in the various hydrated forms thereof, capable of rendering zinc bioavailable and absorbable, preferably in the form of a cation or elementary zinc, by the body once administered thereto.

Unless otherwise specified, the indication that a composition "comprises" one or more components means that other components may also be present in addition to the one or ones specifically indicated, even where they have not necessarily been expressly indicated, i.e. the composition may also contain exclusively those components, and the indication that a composition "consists" of certain components means that the presence of other components is excluded.

Advantageously, the mixture according to the invention and/or the composition according to the invention comprise or consist of natural ingredients, which thus make both the mixture and the pharmaceutical and/or nutraceutical composition comprising that mixture free of side effects and characterized by high tolerability.

In fact, the mixture according to the invention or the composition comprising it does not have any significant adverse effects. Precisely for these reasons, it may be advantageously administered to a broad category of subjects, including more fragile ones, such as the disabled and/or elderly.

The mixture as defined above and a composition comprising said mixture can be advantageously used as a medicament. Said mixture and said composition are useful for reducing and/or treating prostate inflammation and the symptoms associated therewith, preferably for reducing and/or treating benign prostatic hyperplasia and the inflammation associated therewith.

Said prostate inflammation can be chronic prostate inflammation (chronic prostatitis) and said symptoms associated with prostate inflammation are lower urinary tract symptoms (LUTS).

LUTS are classified into:
- symptoms of the bladder filling phase. Fitting into this category are: the need to urinate frequently (pollakiuria), also during the night (nocturia), urgency and urinary incontinence in the most serious cases;
- symptoms of the bladder emptying phase (when urine is expelled). Among these we may mention: urinary hesitancy (difficulty/delay in urinating), a reduced and intermittent urinary stream, burning during urination;
- post-micturition symptoms, which appear after having urinated: post-micturition dribble and the feeling of incomplete emptying.

These disorders may occur individually or be combined in various ways.

Therefore, a third aspect of the present invention relates precisely to the medical use of the mixture according to the first aspect of the invention and/or of the composition of the second aspect of the invention, preferably for reducing and/or treating prostate inflammation and the symptoms associated therewith, more preferably for reducing and/or treating benign prostatic hyperplasia and the inflammation associated therewith.

In the context of the present invention, the term "reducing" or "reduction of" in reference to prostate inflammation is meant to indicate a lowering or decrease in the level of inflammation compared to the initial level (i.e. the level of inflammation before the treatment with the mixture and/or composition according to the invention began).

In the context of the present invention, the term "treating" or "treatment of" in reference to prostate inflammation and benign prostatic hyperplasia is meant to indicate the complete cure from the pathology in question, i.e. absence of prostate inflammation and absence of benign prostatic hyperplasia.

A final aspect of the present description relates to a method for treating prostate inflammation and/or benign prostatic hyperplasia which comprises administering to a subject in need the mixture and/or the composition according to the invention.

In the context of the present invention, the term "subject in need" is meant to indicate a male individual affected by prostate inflammation.

### DESCRIPTION OF THE FIGURES

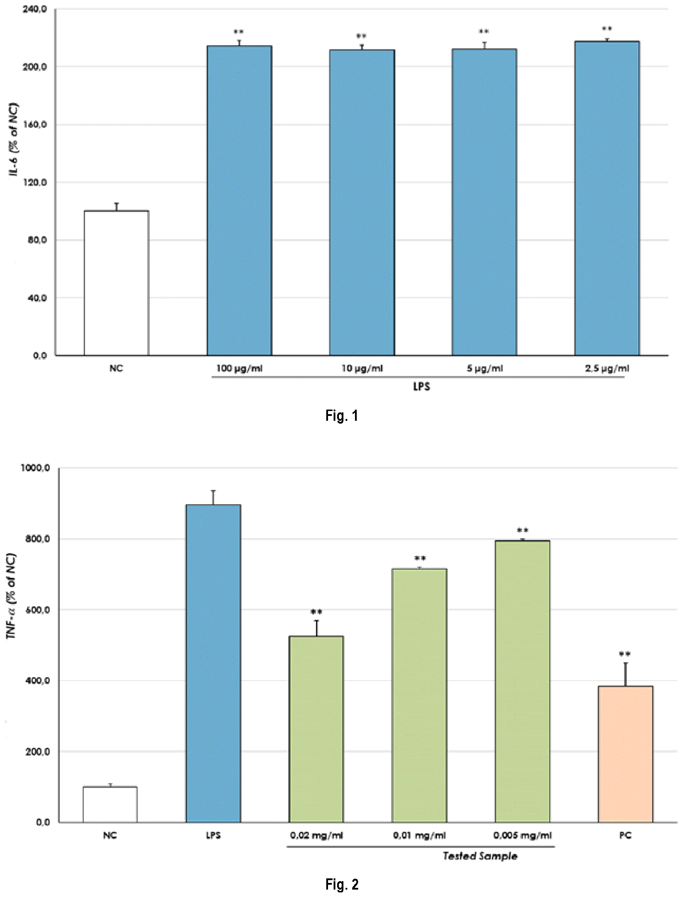

Figure 1 shows the levels of IL-6 in cells stimulated with LPS at different concentrations. The values are expressed as means + standard deviation. Statistical processing of the data was carried out by means of Student's t-test. Values of p<0.05 are considered significant. NC = unstimulated control cells; LPS = lipopolysaccharide; ** p<0.01 vs NC.
Figure 2 shows the levels of TNF-α in cells stimulated with LPS and treated with the mixture according to the invention at different concentrations. The percentages were calculated relative to the negative control cells NC (unstimulated, untreated cells). The values are expressed as means + standard deviation. Statistical processing of the data was carried out by means of Student's t-test. Values of p<0.05 are considered significant. LPS = lipopolysaccharide; PC = positive control (hydrocortisone); **p<0.01 vs LPS.

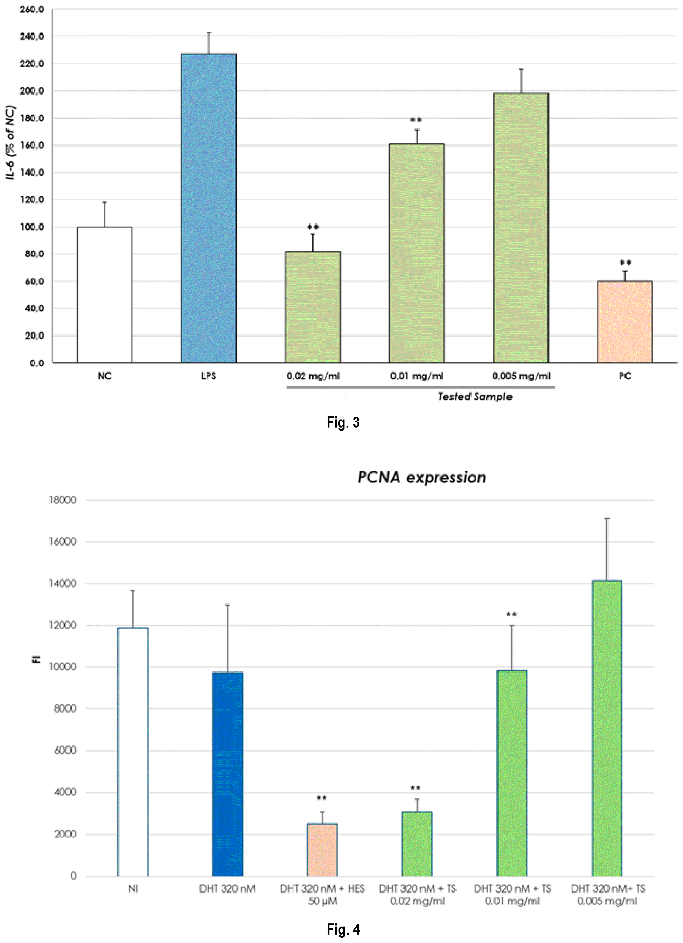

Figure 3 shows the levels of IL-6 in cells stimulated with LPS and treated with the mixture according to the invention at different concentrations. The percentages were calculated relative to the negative control cells NC (unstimulated, untreated cells). The values are expressed as means + standard deviation. Statistical processing of the data was carried out by means of Student's t-test. Values of p<0.05 are considered significant. LPS = lipopolysaccharide; PC = positive control (hydrocortisone); **p<0.01 vs LPS.
Figure 4 shows the PCNA expression levels in cells induced with DHT. The values related to the fluorescence intensity (FI) are expressed as means + standard deviation. Statistical processing of the data was carried out by means of Student's t-test; values of p<0.05 are considered significant. DHT = dihydrotestosterone; HES = hesperidin; TS = Tested Sample, i.e. the mixture tested in the *in vitro* study 2; * p<0.05 vs NI; **p<0.01 vs NI.

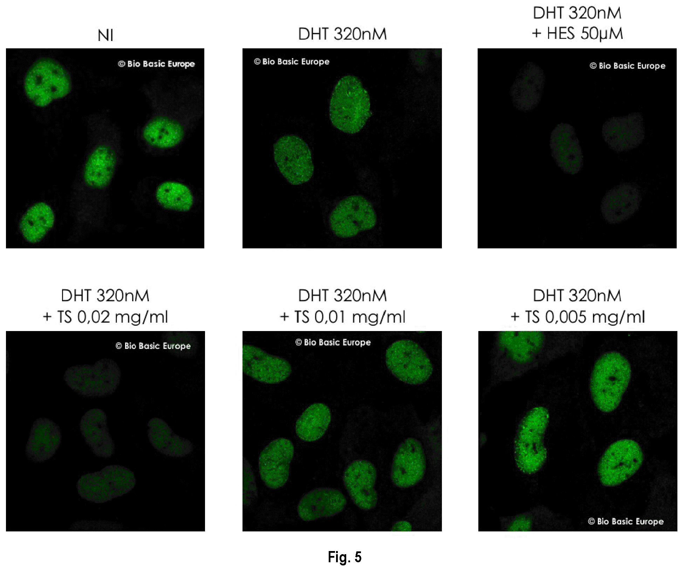

Figure 5 shows the highest fluorescence data for every experimental condition, as described in study 2. The images were acquired with a confocal microscope and processed with ImageJ software; they show the expression of the PCNA protein within the cell nuclei.

### DETAILED DESCRIPTION OF THE INVENTION

A first aspect of the present invention relates to a mixture comprising or, alternatively, consisting of:
i) an epigallocatechin-3-gallate (EGCG) and/or an extract of *Camellia sinensis (L.) Kuntze*;
ii) one or more isoflavones selected in the group comprising or, alternatively, consisting of formononetin, daidzein, genistein, biochanin A and/or an extract of red clover *Trifolium pratense L. Leguminosa*; and
iii) a phenylpropanoid teupolioside and/or an extract of *Ajuga Reptans L.*

Preferably, in combination or association with the components i) + ii) + iii), the mixture further comprises:
iv) selenium or a salt thereof and/or selenium in an organic form, preferably selected in the group comprising or, alternatively, consisting of L-selenomethionine, sodium selenite, sodium selenate, selenocysteine, more preferably L-selenomethionine; and/or
v) zinc or a salt thereof and/or zinc in an organic form, preferably selected in the group comprising or, alternatively, consisting of zinc gluconate, zinc picolinate, zinc acetate, zinc bisglycinate, zinc sulfate and zinc citrate, more preferably zinc citrate and/or mixtures thereof.

The mixture according to the invention comprises i) an epigallocatechin-3-gallate (EGCG) and/or a dry extract of *Camellia sinensis (L.) Kuntze*, in combination or association with said components ii) and iii) and, optionally, with said component iv) and/or v).

Epigallocatechin-3-gallate (EGCG) is a type of catechin and is the most abundant catechin in tea, more preferably in green tea, and it has the molecular formula C₂₂H₁₈O₁₁ and preferably the CAS number 989-51-5.

According to the present invention, said epigallocatechin-3-gallate (EGCG) can be of synthetic and/or semisynthetic origin or derive from a dry extract of green tea (*Camellia sinensis*).

The mixture according to the invention can comprise i) an epigallocatechin-3-gallate (EGCG) of synthetic and/or semisynthetic origin in addition to or as a replacement for an extract of *Camellia sinensis (L.) Kuntze.*

*Camellia sinensis (L.) Kuntze* is a plant of the family *Theaceae*; it is an erect shrub with ovate acuminate leaves and white flowers with numerous stamens of a golden yellow color. All types of tea originate from the processing of leaves, buds and other parts of this plant: only the processing methods differentiate the various types of tea.

Preferably, said i) epigallocatechin-3-gallate (EGCG) is present, in whole or in part, as a phytocomplex; more preferably, said phytocomplex can be present in a dry extract of *Camellia sinensis (L.) Kuntze*.

Said dry extract of *Camellia sinensis (L.) Kuntze* has a titer of epigallocatechin-3-gallate (EGCG) preferably ≥ 13%, preferably comprised from 13% to 40%, preferably from 15% to 30%, more preferably from 19% to 25%, measured by HPLC.

Preferably, the mixture according to the invention comprises a dry extract of *Camellia sinensis (L.) Kuntze* in an amount by weight comprised from 30% to 70%, preferably from 40% to 60%, even more preferably from 45% to 55%, even more preferably 48% or 49% by weight, relative to the total weight of the mixture, in combination or association with said components ii) and iii) and, optionally, with said component iv) and/or v).

Preferably, said i) epigallocatechin-3-gallate (EGCG) and/or said extract of *Camellia sinensis (L.) Kuntze* can be present in the form of a phytosomal solid dispersion, in combination or association with said components ii) and iii) and, optionally, with said component iv) and/or v).

It is well known that active components with a high polarity might not pass across the lipid barrier of the gastrointestinal wall and thus not be absorbed. The phytosomal form, besides ensuring the stability of the extract, improves its bioavailability and efficacy thanks to its ability to pass through lipid-rich biological membranes. At the gastrointestinal level, a phytosome is capable of passing from the outer aqueous phase to the enterocyte, into the lipid phase of the outer membrane, and from here into the cell until reaching the bloodstream.

At the skin level, a phytosome passes through the skin barrier, interacting with its structural components; once it reaches the dermis, it undergoes a slow, progressive decomplexation, releasing the active component in situ.

An example of a commercial product comprising an extract of *Camellia sinensis (L.) Kuntze* in the form of a phytosomal solid dispersion is the product GREENSELECT^{®} Phytosome^{®}_{,} comprising an extract of *Camellia sinensis (L.) Kuntze* in the form of a phytosomal solid dispersion of green tea dry extract, standardized in catechins and decaffeinated, in soy phospholipids in a 1:2 ratio.

Preferably, said product is composed of approximately 33% w/w of decaffeinated green tea dry extract (CAS number for all the extracts of *Camellia sinensis*, e.g. 283-519-7) and approximately 67% w/w of phospholipids (lecithin CAS number, e.g. 8002-43-5).

Said extract is obtained from leaves of *Camellia sinensis (L.) Kuntze* by extraction in water.

Preferably, said product appears as a powder whose granules have the following particle size: < 75 mesh (200 microns): approx. 81%; < 150 mesh (100 microns): approx. 57%; < 300 mesh (50 microns): approx. 37%.

Preferably, the amount of sulfated ash is ≤ 10.0% (Ph. Eur.) and the moisture ≤ 1.0% (K. Fischer; Ph. Eur., met. A). Preferably, the dry extract of *Camellia sinensis (L.) Kuntze* present in said product has a titer of -(-) epigallocatechin-3-O-gallate (EGCG) preferably ≥ 13%, preferably comprised from 13% to 40%, preferably from 15% to 30%, more preferably from 19% to 25%, measured by HPLC and referring to the anhydrous substance free of solvents.

Preferably, said dry extract of *Camellia sinensis (L.) Kuntze* comprises one or more of the following active compounds: flavanols (catechic polyphenols), flavonols and phenolic acids, in combination or association with said components ii) and iii) and, optionally, with said component iv) and/or v).

Said extract of *Camellia sinensis (L.) Kuntze* comprises catechins (expressed as -(-)epigallocatechin-3-O-gallate (EGCG)) preferably ≥ 15%, preferably comprised from 15% to 40%, preferably from 17% to 30%, more preferably from 19% to 25%, measured by HPLC and referring to the anhydrous substance free of solvents, in combination or association with said components ii) and iii) and, optionally, with said component iv) and/or v).

In the context of the present invention, the terms (-)-epigallocatechin-3-O-gallate and epigallocatechin-3-gallate refer to the same molecule, the main antioxidant polyphenol of green tea. Furthermore, the abbreviation EGCG and the name "epigallocatechin-3-gallate" are commonly used as synonyms.

In addition to i) as described above, the mixture according to the invention comprises ii) one or more isoflavones selected in the group comprising or, alternatively, consisting of formononetin, daidzein, genistein, biochanin A and/or an extract of red clover *Trifolium pratense L. Leguminosa*, in combination or association with said component iii) and, optionally, with said component iv) and/or v).

*Trifolium pratense L. Leguminosa,* also called red clover or purple clover, is a perennial herbaceous plant of the family *Fabacee*. Said ii) one or more isoflavones can be of synthetic and/or semisynthetic origin or derive from a dry extract of *Trifolium pratense L. Leguminosa.*

Preferably, the mixture according to the invention comprises ii) one or more isoflavones selected in the group consisting of formononetin, daidzein, genistein, biochanin A and/or an extract of red clover *Trifolium pratense L. Leguminosa*.

The mixture according to the invention can comprise said ii) one or more isoflavones of synthetic and/or semisynthetic origin in addition to or as a replacement for an extract of *Trifolium pratense L. Leguminosa,* in combination or association with said components i) and iii) and, optionally, with said component iv) and/or v).

Preferably, said ii) one or more isoflavones can be present, in whole or in part, as a phytocomplex; more preferably, said phytocomplex can be present in a dry extract of *Trifolium pratense L. Leguminosa,* in combination or association with said components i) and iii) and, optionally, with said component iv) and/or v).

Advantageously, said one or more isoflavones, preferably present in a dry extract of red clover *Trifolium pratense L. Leguminosa,* perform an antioxidant and anti-inflammatory action together with the minerals and vitamins present in said extract. Therefore, the mixture according to the invention comprises preferably a dry extract of *Trifolium pratense L. Leguminosa* comprising one or more isoflavones, preferably formononetin, daidzein, genistein, biochanin A, in combination or association with said components i) and iii) and, optionally, with said component iv) and/or v).

Preferably, said dry extract of *Trifolium pratense L. Leguminosa* has a titer of total isoflavones (formononetin, daidzein, genistein, biochanin A) ≥ 20%, preferably comprised from 20% to 50%, preferably from 20% to 40%, more preferably 20%, measured by HPLC, in combination or association with said components i) and iii) and, optionally, with said component iv) and/or v).

An example of a commercial product comprising an extract of *Trifolium pratense L. Leguminosa* is the product supplied by Farezis Pharma Srl, which appears as a fine brown-colored powder with a particle size of 99% passing through an 80 mesh screen. The extract is obtained from the leaves and flowers of the plant *Trifolium pratense L. Leguminosa*, with ethanol as the extraction solvent. This product has a titer of total isoflavones (formononetin, daidzein, genistein, biochanin A) ≥ 20%, moisture ≤ 5.0%, and the amount of sulfated ash is ≤ 5.0%.

Preferably, the mixture according to the invention comprises a dry extract of *Trifolium pratense L. Leguminosa* in an amount by weight comprised from 30% to 70%, preferably from 35% to 60%, even more preferably from 40% to 50%, even more preferably 41% by weight, relative to the total weight of the mixture, in combination or association with said components i) and iii) and, optionally, with said component iv) and/or v).

The mixture according to the invention, in addition to i) + ii) as described above, comprises iii) a phenylpropanoid teupolioside and/or an extract of *Ajuga Reptans L.*, in combination or association with, optionally, said component iv) and/or v).

*Ajuga Reptans L.,* commonly known as bugle, is a perennial herbaceous plant belonging to the family *Lamiaceae*. Phenylpropanoid teupolioside, also known as lamiuside A, is a secondary metabolite produced by the plant *Ajuga Reptans L*.

The small amount thereof in the plant and its coexistence with other polyphenols make the extraction and purification of this polyphenol difficult to achieve through traditional industrial methods.

Said iii) phenylpropanoid teupolioside can be of synthetic and/or semisynthetic origin or derive from an extract of *Ajuga Reptans L.*

The mixture according to the invention can comprise said iii) phenylpropanoid teupolioside of synthetic and/or semisynthetic origin in addition to or as a replacement for an extract of *Ajuga Reptans L.,* in combination or association with said components i) and ii) and, optionally, with said component iv) and/or v).

Preferably, said iii) phenylpropanoid teupolioside is present, in whole or in part, as a phytocomplex; more preferably, said phytocomplex can be present in a dry extract of *Ajuga Reptans L.*

Preferably, said dry extract of *Ajuga Reptans L.* has a titer of phenylpropanoid teupolioside ≥ 10%, preferably comprised from 10% to 40%, preferably from 15% to 30%, more preferably from 20% to 25%, measured by HPLC, in combination or association with said components i) and ii) and, optionally, with said component iv) and/or v).

Teupol 10P is an example of a commercial product comprising said phenylpropanoid teupolioside, obtained from suspension cell cultures of *Ajuga reptans*, in combination or association with said components i) and ii) and, optionally, with said component iv) and/or v).

Preferably, the mixture according to the invention comprises a dry extract of *Ajuga Reptans L.* in an amount by weight comprised from 2% to 20%, preferably from 5% to 15%, even more preferably from 8% to 10%, even more preferably 8% by weight, relative to the total weight of the mixture, in combination or association with said components i) and ii) and, optionally, with said component iv) and/or v).

In addition to i) + ii) + iii), the mixture according to the invention can further optionally comprise, in association or combination, said component iv) selenium or a salt thereof and/or selenium in an organic form, preferably L-selenomethionine, and/or said component v) zinc or a salt thereof and/or zinc in an organic form, preferably zinc citrate. The selenium salts that can be present in the mixture according to the invention comprising the components i) + ii) + iii) are selected from the group comprising or, alternatively, consisting of L-selenomethionine, sodium selenite, sodium selenate, selenocysteine, more preferably L-selenomethionine.

An example of L-selenomethionine in the mixture according to the invention is present in the product having the trade name "Selenium L-methionine 0.5%", supplied by Farezis Pharma Srl.

This product appears as a white-colored powder and comprises 1.25% to 1.35% L-selenomethionine (CAS number 3211-76-5), of which 0.5% to 0.55% selenium.

Said product has a loss on drying ≤ 5.0% and bulk density comprised from 0.6 g/ml a 1.2 g/ml. The tapped density is comprised from 0.9 to 1.5 g/ml.

An example of zinc citrate in the mixture according to the invention is present in the product having the trade name "Zinc Citrate 3-Hydrate", supplied by Dr. Paul Lohmann GmbH & Co. KGaA.

The product appears as a white-colored powder, has a pH value comprised from 5 to 7 and has a zinc content comprised from 30% to 35%, more preferably from 31% to 32.5%.

In combination/association with or as a replacement for iv) selenium or a salt thereof and/or selenium in an organic form, as described above, the mixture according to the invention comprises zinc or zinc salts and/or zinc in an organic form selected in the group comprising or, alternatively, consisting of zinc gluconate, zinc picolinate, zinc acetate, zinc bisglycinate, zinc sulfate and zinc citrate, more preferably zinc citrate.

More preferably, the mixture according to the invention, in addition to i) + ii) + iii), can further optionally comprise, in combination or association, both iv) selenium or salts thereof and/or selenium in an organic form, preferably L-selenomethionine, and v) zinc or salts thereof and/or zinc in an organic form, preferably zinc citrate.

More preferably, the mixture comprises or, alternatively, consists of:
i) an epigallocatechin-3-gallate (EGCG) and/or a dry extract of *Camellia sinensis (L.) Kuntze*;
ii) one or more isoflavones selected in the group comprising or, alternatively, consisting of formononetin, daidzein, genistein, biochanin A and/or a dry extract of *Trifolium pratense L. Leguminosa*;
iii) a phenylpropanoid teupolioside and/or a dry extract of *Ajuga Reptans L*;
iv) selenium or salts thereof and/or selenium in an organic form; and/or
v) zinc or salts thereof and/or zinc in an organic form.

Even more preferably, the mixture comprises or, alternatively, consists of:
i) an epigallocatechin-3-gallate (EGCG) and/or a dry extract of *Camellia sinensis (L.) Kuntze*;
ii) one or more isoflavones selected in the group comprising or, alternatively, consisting of formononetin, daidzein, genistein, biochanin A and/or a dry extract of *Trifolium pratense L. Leguminosa*;
iii) a phenylpropanoid teupolioside and/or a dry extract of *Ajuga Reptans* L;
iv) L-selenomethionine; and/or
v) zinc citrate.

Preferably, said iv) selenium or a salt thereof, preferably L-selenomethionine, optionally present in the mixture according to the invention in combination or association with the components i) + ii) + iii), is present in the mixture according to the invention in an amount by weight, relative to the total weight of the mixture, preferably comprised from 0.007% to 0.02%, preferably from 0.008% to 0.01%, more preferably from 0.009% to 0.01%.

Preferably, said v) zinc or a salt thereof, preferably zinc citrate, is present in the mixture according to the invention in an amount by weight, relative to the total weight of the mixture, preferably comprised from 0.5% to 10%, preferably from 1% to 5%, more preferably from 2% to 4%.

Even more preferably, the mixture according to the invention comprises or, alternatively, consists of:
i) an extract of *Camellia sinensis (L.) Kuntze;*
ii) an extract of *Trifolium pratense L. Leguminosa;*
iii) an extract of *Ajuga Reptans L.;*
iv) selenium; and
v) zinc.

Even more preferably, the mixture according to the invention comprises or, alternatively, consists of:
i) an extract of *Camellia sinensis (L.) Kuntze;*
ii) an extract of *Trifolium pratense L. Leguminosa;*
iii) an extract of *Ajuga Reptans L.;*
iv) a selenium salt, preferably L-selenomethionine; and
v) a zinc salt, preferably zinc citrate.

Preferably, the mixture according to the invention comprises or, alternatively, consists of:
i) an extract of *Camellia sinensis (L.) Kuntze* (titer ≥ 13%), preferably in the form of a phytosomal solid dispersion, in an amount by weight, relative to the total weight of the mixture, preferably comprised from 30% to 70%, preferably from 40% to 60%, more preferably from 45% to 55%;
ii) an extract of *Trifolium pratense L. Leguminosa* (titer ≥ 20%), comprising or, alternatively, consisting of formononetin, daidzein, genistein, biochanin A, in an amount by weight, relative to the total weight of the mixture, preferably comprised from 30% to 70%, preferably from 35% to 60%, more preferably from 40% to 50%;
iii) an extract of *Ajuga Reptans L.* (titer ≥ 10%), in an amount by weight, relative to the total weight of the mixture, preferably comprised from 2% to 20%, preferably from 5% to 15%, more preferably from 8% to 10%;
iv) selenium in an amount by weight, relative to the total weight of the mixture, preferably comprised from 0.007% to 0.02%, preferably from 0.008% to 0.01%, more preferably from 0.009% to 0.01%; and/or
v) zinc in an amount by weight, relative to the total weight of the mixture, preferably comprised from 0.5% to 10%, preferably from 1% to 5%, more preferably from 2% to 4%.

Furthermore, preferably, the mixture according to the invention specified above comprises an extract of *Trifolium pratense L. Leguminosa* (titer ≥ 20%), consisting of formononetin, daidzein, genistein, biochanin A, in an amount by weight, relative to the total weight of the mixture, preferably comprised from 30% to 70%, preferably from 35% to 60%, more preferably from 40% to 50%.

More preferably, the mixture according to the invention comprises or, alternatively, consists of:
i) an extract of *Camellia sinensis (L.) Kuntze* (titer ≥ 13%), preferably in the form of a phytosomal solid dispersion, in an amount by weight, relative to the total weight of the mixture, of 48% or 49%;
ii) an extract of *Trifolium pratense L. Leguminosa* (titer ≥ 20%) comprising or, alternatively, consisting of formononetin, daidzein, genistein, biochanin A in an amount by weight, relative to the total weight of the mixture, of 41%;
iii) an extract of *Ajuga Reptans L.* (titer ≥ 10%) in an amount by weight, relative to the total weight of the mixture, of 8%;
iv) selenium in an amount by weight, relative to the total weight of the mixture, of 0.009%; and/or
v) zinc in an amount by weight, relative to the total weight of the mixture, of 2.4%.

The mixture as described above can be advantageously used as a medicament.

Advantageously, the mixture and the composition comprising said mixture, both the subject-matter of the present invention, have a valid application in subjects with a high PSA value because by reducing solely the inflammatory component, responsible for such a high PSA value, it is capable of acting only on the physiological component of the PSA. In practical terms, if a subject with a high PSA value is treated with the mixture and/or composition of the present invention and after said treatment the PSA value is reduced but still remains at values above the allowed limits, there is a clear indication that the subject has a neoplasia that must be treated immediately without delay, pharmacologically or surgically. If, by contrast, the mixture and/or composition of the present invention drastically reduce the PSA value, there is a risk of conveying a misleading message that the problem has been solved when the problem instead persists, because a neoplasia is present which must be treated without delay.

Preferably, said mixture is for use in a method, therapeutic or non-therapeutic, for reducing and/or treating prostate inflammation and the symptoms associated therewith, and/or in a method for reducing and/or treating benign prostatic hyperplasia. Preferably, said mixture is for use in a method, therapeutic or non-therapeutic, for reducing and/or treating chronic prostate inflammation (chronic prostatitis).

Preferably, said mixture is capable of reducing and/or treating symptoms associated with prostate inflammation, such as lower urinary tract symptoms (LUTS) selected from the group comprising or, alternatively, consisting of: pollakiuria, nocturia, urinary urgency and incontinence, urinary hesitancy (difficulty/delay in urinating), a reduced and intermittent urinary stream, burning during urination, and feeling of incomplete emptying.

More preferably, said mixture is for use in a method, therapeutic or non-therapeutic, for reducing and/or treating benign prostatic hyperplasia.

A second aspect of the present invention relates to a composition comprising the mixture according to the invention and, optionally, acceptable food or pharmaceutical grade excipients and/or additives.

The acceptable food or pharmaceutical grade excipients and/or additives present in the composition can be selected from all the substances known to the person skilled in the art of pharmaceutical technology or food preparations.

The types of food, nutraceutical and/or pharmaceutical additives used for the preparation of the compositions of the invention, the ratios between the contents of additives and active ingredients and the methods for preparing the food, nutraceutical and/or pharmaceutical composition can be appropriately selected by the person skilled in the art.

By way of example, the composition according to the invention can comprise silica, calcium phosphate and magnesium stearate as excipients.

The composition can further contain sweetening agents, preservative agents and flavoring agents.

The composition of the invention is a pharmaceutical and/or nutraceutical composition for oral use, preferably in the form of a capsule, a tablet, a sachet, a stick, an orodispersible stick, a gel for oral use or granules.

More preferably, said composition is in the form of a capsule, even more preferably a delayed-release capsule. Even more preferably, said delayed-release capsule can be an acid-resistant capsule that dissolves as a function of pH in order to release the composition of the invention at the intestinal level.

Preferably, said capsule comprising the composition according to the invention is an HPMC- and pectin-based capsule. Advantageously, said HPMC- and pectin-based capsule protects the composition according to the invention from the conditions of the upper gastrointestinal tract: in particular from the acidity of the stomach pH and exposure to bile salts in the small intestine.

An example of an HPMC- and pectin-based capsule is represented by the EMBOCAPS ^{®} AP60 capsule.

The formulation of the EMBOCAPS^{®} AP60 capsule resists disintegration in an acidic environment for 60 minutes without the need for further coating processes, shellac or phthalates. EMBOCAPS^{®} AP protects its dosage from acid by delaying the release of the capsule in the stomach and safely distributing it to the intestinal tract.

Preferably, the composition is in the form of an HPMC- and pectin-based capsule, which is an acid-resistant capsule that dissolves as a function of pH in order to release the composition of the invention at the intestinal level.

Preferably, the composition of the invention comprises or, alternatively, consists of:
- from 100 to 500 mg, preferably from 150 to 400 mg, even more preferably from 200 to 350 mg, even more preferably 300 mg of dry extract of *Camellia sinensis (L.) Kuntze* in the form of a phytosomal solid dispersion preferably having a titer of epigallocatechin-3-gallate (EGCG) ≥ 13%, preferably comprised from 13% to 40%, preferably from 15% to 30%, more preferably from 19% to 25%, measured by HPLC;
- from 100 to 500 mg, preferably from 150 to 400 mg, even more preferably from 200 to 350 mg, even more preferably 250 mg of a dry extract of *Trifolium pratense L. Leguminosa* preferably having a titer of total isoflavones (formononetin, daidzein, genistein, biochanin A) ≥ 20%, preferably comprised from 20% to 50%, preferably from 20% to 40%, even more preferably 20%, measured by HPLC;
- from 1 to 200 mg, preferably from 10 to 100, more preferably from 20 to 50 mg, even more preferably 50 mg of a dry extract of *Ajuga Reptans L.,* preferably having a titer of phenylpropanoid teupolioside ≥ 10%, preferably comprised from 10% to 40%, preferably from 15% to 30%, more preferably from 20% to 25%, measured by HPLC;
- from 45 to 65 µg, preferably from 50 to 60 µg, more preferably from 55 to 58 µg, even more preferably 55 µg of selenium or salts thereof and/or selenium in an organic form;
- from 1 to 40 mg, preferably from 5 to 30 mg, more preferably from 10 to 20 mg, even more preferably 15 mg of zinc or salts thereof and/or zinc in an organic form and,
optionally, acceptable food or pharmaceutical grade excipients and/or additives.

The composition according to the invention can comprise a dry extract of *Camellia sinensis (L.) Kuntze* (green tea) not in the form of a phytosomal solid dispersion. Preferably, the composition can comprise from 70 mg to 150 mg of a dry extract of *Camellia sinensis (L.) Kuntze* (green tea) not in the form of a phytosomal solid dispersion, preferably from 80 mg to 120 mg, more preferably from 90 mg to 110 mg. Even more preferably, the composition comprises 99 mg of dry extract of *Camellia sinensis (L.) Kuntze* (green tea) (see the composition in Table 2).

Therefore, the composition comprises or, alternatively, consists of:
- from 70 to 150 mg, preferably from 80 to 120 mg, even more preferably from 90 to 110 mg, even more preferably 99 mg of dry extract of *Camellia sinensis (L.) Kuntze* (green tea) not in the form of a phytosomal solid dispersion, wherein said dry extract preferably has a titer of epigallocatechin-3-gallate (EGCG) ≥ 13%, preferably comprised from 13% to 40%, preferably from 15% to 30%, more preferably from 19% to 25%, measured by HPLC;
- from 100 to 500 mg, preferably from 150 to 400 mg, even more preferably from 200 to 350 mg, even more preferably 250 mg of a dry extract of *Trifolium pratense L. Leguminosa* preferably having a titer of total isoflavones (formononetin, daidzein, genistein, biochanin A) ≥ 20%, preferably comprised from 20% to 50%, preferably from 20% to 40%, even more preferably 20%, measured by HPLC;
- from 1 to 200 mg, preferably from 10 to 100, more preferably from 20 to 50 mg, even more preferably 50 mg of a dry extract of *Ajuga Reptans L.,* preferably having a titer of phenylpropanoid teupolioside ≥ 10% preferably comprised from 10% to 40%, preferably from 15% to 30%, more preferably from 20% to 25%, measured by HPLC;
- from 45 to 65 µg, preferably from 50 to 60 µg, more preferably from 55 to 58 µg, even more preferably 55 µg of selenium or salts thereof and/or selenium in an organic form;
- from 1 to 40 mg, preferably from 5 to 30 mg, more preferably from 10 to 20 mg, even more preferably 15 mg of zinc or salts thereof and/or zinc in an organic form; and,
optionally, acceptable food or pharmaceutical grade excipients and/or additives.

The composition as described above can be advantageously used as a medicament.

Preferably, said composition is for use in a method, therapeutic or non-therapeutic, for reducing and/or treating prostate inflammation and the symptoms associated therewith, and/or in a method for reducing and/or treating benign prostatic hyperplasia. Preferably, said mixture is for use in a method, therapeutic or non-therapeutic, for reducing and/or treating chronic prostate inflammation (chronic prostatitis).

Preferably, said composition is capable of reducing and/or treating symptoms associated with prostate inflammation, such as lower urinary tract symptoms (LUTS) selected from the group comprising or, alternatively, consisting of: pollakiuria, nocturia, urinary urgency and incontinence, urinary hesitancy (difficulty/delay in urinating), a reduced and intermittent urinary stream, burning during urination, and feeling of incomplete emptying.

As shown in the experimental section, in fact, the mixture according to the invention is capable of reducing the level of IL-6 and TNFα, proinflammatory cytokines released in the event of prostate inflammation and which, by acting as mitogenic stimuli, trigger the proliferation of the epithelial component, bringing about an overall volumetric increase of the organ (see study 1). Furthermore, the Applicant has observed that the mixture according to the invention is capable of significantly reducing the expression of PCNA (proliferating cell nuclear antigen) in a cell model of benign prostatic hypertrophy (see study 2).

More preferably, said mixture is for use in a method, therapeutic or non-therapeutic, for reducing and/or treating benign prostatic hyperplasia.

The mixture according to the first aspect of the invention and/or composition in accordance with the second aspect of the invention can be advantageously used as adjuvants of drugs and/or dietary supplements conventionally used to reduce and/or treat prostate inflammation and/or benign prostatic hyperplasia.

Said mixture and/or said composition can be advantageously used in a method of treatment, therapeutic or non-therapeutic, of a prostate inflammation (including chronic inflammation, or chronic prostatitis) and of the symptoms associated therewith, such as the lower urinary tract symptoms (LUTS) specified above, and/or in a method for reducing and/or treating benign prostatic hyperplasia which comprises administering the mixture and/or the composition according to the invention to a subject in need.

In said method of treatment, the administration of the mixture and/or of the composition as described in the present patent application can take place before or after or in concomitance with at least one drug conventionally used in the treatment of a prostate inflammation and/or benign prostatic hyperplasia, and/or before or after or in concomitance with dietary supplements.

Preferably, said method of treatment comprises administering said composition, preferably in the form of a capsule, even more preferably in the form of an HPMC- and pectin-based capsule. More preferably, said method of treatment comprises administering a capsule having the composition specified in example 1 once a day, preferably on an empty stomach. The method of use of the composition of the invention is used will depend on the degree of severity of the prostate inflammation and/or benign prostatic hyperplasia and/or the state of health of the subject in need and may be established by physicians, according to needs.

### EXAMPLES

### Example 1

Composition in the form of an HPMC- and pectin-based capsule containing:

**Table 1**

| **Components** | **Dosage form** |
|---|---|
| dry extract of *Camellia sinensis (L.) Kuntze* (titer ≥ 13%) | 300 mg |
| comprising epigallocatechin-3-gallate (EGCG) | 39 mg |
| dry extract of *Trifolium pratense L.* (titer 20%) | 250 mg |
| comprising isoflavones | 50 mg |
| dry extract of *Ajuga Reptans L.* (titer 10%) comprising | 50 mg |
| phenylpropanoid teupolioside | 5 mg |
| Selenium | 55 µg |
| from L-selenomethionine | |
| Zinc | 15 mg |
| from zinc citrate | |

and, optionally, food or pharmaceutical grade excipients and/or additives.

For example, said dry extract of *Camellia sinensis (L.) Kuntze* (titer ≥ 13%) comprising epigallocatechin-3-gallate (EGCG) in the form of a phytosomal solid dispersion is GREENSELECT^{®} Phytosome^{®}.

For example, said dry extract of *Ajuga Reptans L.* (titer 10%) comprising phenylpropanoid teupolioside is Teupol 10P^{®}. By way of example, the composition can comprise silica, calcium phosphate and magnesium stearate as excipients.

### Example 2

Composition in the form of a gastro-resistant capsule containing:

**Table 2**

| **Components** | **Dosage form per daily (max) dose (1 capsule)** | **% NRV (daily nutrient reference value - Reg. (EU) 1169/2011)** | **Per 100 g** |
|---|---|---|---|
| GREENSELECT^{®} Phytosome^{®} | 300 mg | | 36.144 g |
| Green tea | 99 mg | | 11.93 g |
| - of which catechins | 57 mg | | 6.87 g |
| - of which epigallocatechin-3-gallate (EGCG) | 39 mg | | 4.7 g |
| Dry extract of *Trifolium pratense L.* (titer 20%) | 250 mg | | 30.120 g |
| | 50 mg | | 6.024 g |
| - of which isoflavones | | | |
| Teupol 10P^{®} Ajuga *Reptans L.* d.e. from cell culture | 50 mg | | 6.024 g |
| | 5 mg | | 602.4 mg |
| - of which teupolioside | | | |
| Selenium or salts thereof and/or selenium in an organic form | 55 µg | | 6.626 mg |
| Zinc or salts thereof and/or zinc in an organic form | 15 mg | | 1.807 g |

and, optionally, food or pharmaceutical grade excipients and/or additives.

By way of example, the composition can comprise silica, calcium phosphate and magnesium stearate as excipients.

### In vitro studies

The Applicant carried out the following *in vitro* studies with the aim of testing the effectiveness of the mixture according to the invention on a prostate cell model:
1. Evaluation of the ability of the sample (mixture according to the invention) to modulate the expression of pro-inflammatory cytokines (TNF-α and IL-6) in a prostate cell model (anti-inflammatory activity); and
2. Evaluation of the ability of the sample (mixture according to the invention) to modulate cell proliferation in a prostate cell model (antiproliferative activity).

Said studies were carried out by testing the mixture according to the invention as specified in Table 1, comprising the active ingredients present in the extracts, on the WPMY-1 cell line (human prostatic stromal myofibroblast cell line), prostate epithelial cells which can be induced so as to obtain an intracellular microenvironment comparable to inflammatory prostatitis and/or benign prostatic hypertrophy. The experiments were not carried out including the excipient component. Solubility tests were also conducted with an eye to evaluating the impact of the mixture on the viability of the cell model in order to identify the optimal conditions for proceeding with the *in vitro* experiments, described below.

### Detailed description of the studies

### 1. EVALUATION OF THE ABILITY OF A SAMPLE (MIXTURE ACCORDING TO THE INVENTION) TO MODULATE THE EXPRESSION OF PRO-INFLAMMATORY CYTOKINES IN A PROSTATE CELL MODEL

The aim of the test is to evaluate the ability of the tested sample to modulate the expression of pro-inflammatory cytokines in a prostate cell model.

For the test, use was made of WPMY-1 normal human prostate cells, which were stimulated with lipopolysaccharide (LPS) in order to trigger an inflammatory process characterized by an increase in the expression of pro-inflammatory cytokines. First a cell viability assay (MTT) was performed in order to identify the three highest non-cytotoxic concentrations to be used in the subsequent test steps.

The test was performed on different groups of cells: one group was not stimulated (baseline situation), one group was stimulated with LPS and not treated (negative control), and one group was stimulated with LPS and treated with 3 concentrations of the tested sample. After 24 hours of contact, the liquids were collected in order to carry out an assay of TNF-α and IL-6 via the ELISA (enzyme-linked immunosorbent assay) technique.

A reduction in the cytokine levels in the cells treated with the sample compared to untreated cells is indicative of the ability of the sample to modulate inflammatory processes in human prostate cells.

### 2. EVALUATION OF THE ABILITY OF A SAMPLE (MIXTURE ACCORDING TO THE INVENTION) TO MODULATE CELL PROLIFERATION IN A PROSTATE CELL MODEL

The aim of the test is to evaluate the ability of the tested sample to modulate proliferative activity in a cell model of benign prostatic hypertrophy. For the test, use was made of WPMY-1 normal human prostate cells, which were stimulated with dihydrotestosterone (DHT) in order to induce an intracellular microenvironment comparable to a situation of benign prostatic hypertrophy (BPH). The proliferating cell nuclear antigen (PCNA) was considered as a marker of proliferation.

First a cell viability assay (MTT) was performed in order to identify three highest non-cytotoxic concentrations to be used in the subsequent test steps.

The cells were seeded on slides, incubated until the formation of a monolayer and then treated with 3 different concentrations of the sample, chosen based on the results of the preliminary cytotoxicity. The cells were then induced with DHT. One series of cells was not induced and was not treated (negative control). Of the induced cells, one series was not treated. After 24 hours the cells were fixed in methanol, subjected to immunolabeling with an anti-PCNA primary antibody and subsequently with a fluorophore-conjugated secondary antibody. The nuclei were counterstained with a fluorescent nuclear dye. The slides were then mounted so as to be observed with a fluorescence microscope, from which images were acquired, on which, with special software, a quantitative analysis of PCNA was performed.

A reduction in PCNA expression in the cells treated with the sample compared to NC is indicative of an effectiveness of the sample in reducing cell proliferation. The results are supported by the images acquired with the fluorescence microscope.

### Preliminary results

The preliminary results of these studies confirmed the anti-inflammatory and anti-proliferative activity of the mixture according to the invention and hence its effectiveness in reducing and/or treating prostate inflammation and reducing the proliferative activity of epithelial and stromal prostate cells which characterizes benign prostatic hyperplasia.

### Study 1: Evaluation of the ability of the mixture to modulate the expression of pro-inflammatory cytokines in a prostate cell model

The aim of the test is to evaluate the ability of the tested sample (mixture according to the invention) to modulate the expression of the pro-inflammatory cytokines TNF-α and IL-6 in a prostate cell model.

The main culprits in the internal imbalance of the prostate are stromal cells, which normally have the task of sustaining the structure of the gland. The buildup of a phlogistic infiltrate - consisting of T lymphocytes, macrophages and, in acute situations, neutrophilic granulocytes - actively promotes processes of tissue remodeling, fibrosis and hypertrophy of the stroma, thereby compromising the functionality of the lower urinary tract (LUTS). The induction of this immunological microenvironment stimulates the secretion of pro-inflammatory cytokines, specifically IL-6 and TNFα. By acting as mitogenic stimuli, these biochemical mediators trigger the proliferation of the epithelial component, bringing about an overall volumetric increase of the organ.

### 1. Materials and methods

### Cell cultures

The test was conducted on a normal human prostate cell line (WPMY-1), cultured in DMEM (Dulbecco's Modified Eagle Medium) containing 5% fetal bovine serum (FBS) and 1% antibiotics (penicillin and streptomycin) and incubated under standard culture conditions (37°C, 5% CO2).

### Setting up of cell stimulation conditions

In order to induce a state of irritation comparable to prostatitis, WPMY-1 cells were stimulated with lipopolysaccharide (LPS). The cells were seeded in 96-well plates at a density of 1.0 x 10⁴ cells/well, incubated overnight and stimulated with LPS at concentrations of 100, 10, 5 and 2.5 µg/ml for 24 hours. In order to confirm the effectiveness of the stimulation, after 24 of exposure to LPS, the culture media were collected and an assay was performed for IL-6, a pro-inflammatory cytokine whose elevated levels are associated with inflammatory states involving the prostate. An increase in the levels of IL-6 in cells treated with LPS compared to untreated ones thus represents an indicator of the presence of an inflammatory response compatible with an *in vitro* model of prostatitis. The quantification of IL-6 was carried out by ELISA (Enzyme-Linked Immunosorbent Assay).

### Sample and positive control preparation

The mixture tested in the study 1 is: GREENSELECT^{®} PHYTOSOME 9 grams, red clover 7.50 grams, Teupol 10 P 1.50 grams, L-selenomethionine 0.33 grams, zinc citrate 1.44 grams.

The mixture was found to be soluble in DMSO at a concentration of 2.0 mg/ml. Said mixture was then diluted in the cell culture medium until reaching the final concentrations to be used in the tests. Hydrocortisone, a substance with known anti-inflammatory properties, capable of reducing the production of pro-inflammatory cytokines in cell models of inflammation, was used as a positive control. The hydrocortisone was used at a concentration of 1.5 x 10-3 mg/ml.

### 2. Experimental protocol

### Preliminary cytotoxicity assays

Preliminary cytotoxicity assays were performed both to identify the non-cytotoxic concentrations of the mixture to be used in the subsequent test steps and to verify that the LPS used for stimulation did not have cytotoxic effects.

WPMY-1 cells were seeded in 96-well plates at a density of 1.0 x 10⁴ cell/well, incubated overnight and then treated for 24 hours with different concentrations of the substances under examination (mixture and LPS). In order to prevent the solvent from interfering with the test result, the substances (mixture and LPS) dissolved in DMSO were diluted 100 times in culture medium before being administered to the cells.

Cell viability was assessed by means of an assay with MTT [3-(4,5-dimethylthiazol-2-yl)-2,5-diphenyltetrazolium bromide], a yellow-colored tetrazolium compound which is reduced by cells into purple-colored formazan. This conversion is caused by the NADPH or NADH produced by the dehydrogenase present in metabolically active cells. The cells were treated with MTT (1 mg/ml) and incubated for 3 hours under standard conditions. At the end of this period, the MTT solution was eliminated and 100 µl of isopropanol were added in each well to dissolve the formazan crystals formed. The absorbance (optical density, OD) was determined by means of a spectrophotometer reading at a wavelength of 570 nm.

### Evaluation of TNF-α and IL-6 modulation

The WPMY-1 cells were seeded in 96-well plates at a density of 1.0 x 10⁴ cell/well, incubated overnight, then stimulated with LPS (10 µg/ml) and treated with hydrocortisone (1.5 x 10-3 mg/ml) or with the mixture according to the invention (at concentrations chosen based on the result of the preliminary cytotoxicity assay). The cells were then incubated for 24 hours.

The next day the supernatants were collected for the assay of TNF-α and IL-6 by ELISA.

### 3. Results

### Preliminary evaluations of cell viability

The mixture was dissolved in DMSO (2.0 mg/ml), diluted in cell culture medium and tested starting from a concentration of 0.02 mg/ml and with successive 1:2 dilutions (again in cell culture medium). At the tested concentrations, no significant changes in cell viability were observed; therefore, in the subsequent test steps it was decided to use the concentrations of 0.02, 0.01 and 0.005 mg/ml. The concentrations of the single components in the tested dilutions are indicated below in Table 2.1. LPS, tested starting from 100 µM, did not bring about any significant changes in cell viability; therefore, the concentrations of 100, 10, 5 and 2.5 µM were tested for the purpose of setting up the stimulation conditions.

**Table 2.1: Concentrations of the single components in the tested dilutions**

| | **TESTED CONCENTRATION** | | |
|---|---|---|---|
| | **0.02 mg/ml** | **0.01 mg/ml** | **0.005 mg/ml** |
| **GREENSELECT^{®} PHYTOSOME** | 9.10 | 4.55 | 2.28 |
| **Red clover** | 7.59 | 3.79 | 1.90 |
| **Teupol 10P** | 1.52 | 0.76 | 0.38 |
| **L-selenomethionine** | 0.33 | 0.17 | 0.08 |
| **Zinc citrate** | 1.46 | 0.73 | 0.36 |

| | **NC** | **Sample concentration (mg/ml)** | | | | | | | |
|---|---|---|---|---|---|---|---|---|---|
| | | **0.0002** | **0.0003** | **0.0006** | **0.0013** | **0.0025** | **0.005** | **0.01** | **0.02** |
| **Mean OD** | 1.005 | 1.080 | 1.076 | 1.025 | 1.009 | 1.059 | 1.048 | 1.052 | 0.936 |
| **Viability (%)** | 100.0 | 107.5 | 107.1 | 102.0 | 100.5 | 105.5 | 104.3 | 104.7 | 93.2 |

**Table 3: Cell viability**

| | **NC** | **LPS concentration (µg/ml)** | | | |
|---|---|---|---|---|---|
| | | **2.5** | **5.0** | **10.0** | **100.0** |
| **Mean OD** | 1.263 | 1.260 | 1.301 | 1.284 | 1.270 |
| **Viability (%)** | 100.0 | 99.7 | 103.0 | 101.6 | 100.5 |

The absorbance (OD) measured at 570 nm is proportional to cell viability. The percentages shown in the table were calculated based on OD values at 570 nm, considering the OD of the negative control as 100%. NC = negative control (untreated cells).

### Evaluation of the stimulation of WPMY-1 cells to induce a state of irritation

In the presence of LPS, one observes a significant increase in IL-6, which means that the WPMY-1 cells are in a state of irritation comparable to prostatitis (Table 4, Figure 1). No significant differences are observed between the different tested concentrations of LPS.

In the subsequent test steps, the LPS concentration of 10 µg/ml will be used to stimulate the cells.

**Table 4: levels of IL-6 in cells stimulated with LPS at different concentrations.**

| | NC | LPS | | | |
|---|---|---|---|---|---|
| | | 100 µg/ml | 10 µg/ml | 5 µg/ml | 2.5 µg/ml |
| IL-6 (pg/ml) | 80.9 | 173.7 | 171.2 | 172.0 | 176.0 |
| % of NC | 100.0 | 214.7 | 211.6 | 212.5 | 217.5 |
| Change (% of NC) | - | +114.7 | +111.6 | +112.5 | +117.5 |

Absorbance measured at 450 nm is directly proportional to the amount of IL-6 produced by the cells. In order to calculate the concentrations, the OD values obtained were interpolated in a standard curve of IL-6. The percentages were calculated relative to unstimulated cells (NC). The values are expressed as means + standard deviation. Statistical processing of the data was carried out by means of Student's t-test. Values of p<0.05 are considered significant. NC = unstimulated control cells; LPS = lipopolysaccharide; ** p<0.01 vs NC.

### Evaluation of the expression of TNF-α

In cells stimulated with LPS one observes an increase in TNF-α, indicative of an increase in the state of inflammation comparable to prostatitis (Table 5, Figure 2). The tested mixture significantly reduces the TNF-α levels of the stimulated cells. The effect was observed at all the tested concentrations; in particular, compared to the stimulated and untreated cells, a significant reduction in TNF-α of 41.5%, 20.2% and 11.4%, respectively, is noted at the tested concentrations of 0.02, 0.01 and 0.005 mg/ml.

**Table 5: levels of TNF-α in cells stimulated with LPS and treated with the mixture according to the invention at different concentrations.**

| | NC | LPS | LPS | | | |
|---|---|---|---|---|---|---|
| | | | + Mixture | | | + PC |
| | | | 0.02 mg/ml | 0.01 mg/ml | 0.005 mg/ml | |
| TNF-α (pg/ml) | 3.0 | 26.9 | 15.7 | 21.5 | 23.8 | 11.5 |
| % of NC | 100.0 | 896.3 | 524.6 | 715.1 | 794.4 | 383.7 |
| Change (% of LPS) | - | - | -41.5 | -20.2 | -11.4 | -57.2 |

Absorbance measured at 450 nm is directly proportional to the amount of IL-6 produced by the cells. In order to calculate the concentrations, the OD values obtained were interpolated in a standard curve of IL-6. The percentages were calculated relative to the negative control cells NC (unstimulated cells). The values are expressed as means + standard deviation. Statistical processing of the data was carried out by means of Student's t-test.

Values of p<0.05 are considered significant. LPS = lipopolysaccharide; PC = positive control (hydrocortisone); **p<0.01 vs LPS.

### Evaluation of the expression of IL-6

In cells stimulated with LPS an increase in IL-6 is observed, indicating an increase in the state of inflammation comparable to prostatitis. The mixture significantly reduces the IL-6 levels of the stimulated cells. The effect is particularly evident and statistically significant at concentrations of 0.02 and 0.01 mg/ml, with a reduction in IL-6 compared to untreated cells of 64.1% and 29.2%, respectively.

**Table 6: levels of IL-6 in cells stimulated with LPS and treated with the mixture according to the invention at different concentrations.**

| | NC | LPS | LPS | | | |
|---|---|---|---|---|---|---|
| | | | + Mixture | | | + PC |
| | | | 0.02 mg/ml | 0.01 mg/ml | 0.005 mg/ml | |
| IL-6 (pg/ml) | 53.2 | 120.9 | 43.4 | 85.6 | 105.4 | 32.0 |
| % of NC | 100.0 | 227.4 | 81.7 | 161.0 | 198.2 | 60.1 |
| Change (% of LPS) | - | - | -64.1 | -29.2 | -12.9 | -73.6 |

Absorbance measured at 450 nm is directly proportional to the amount of IL-6 produced by cells. In order to calculate the concentrations, the OD values obtained were interpolated in a standard curve of IL-6. The percentages were calculated relative to the negative control cells NC (unstimulated cells). The values are expressed as means + standard deviation. Statistical processing of the data was carried out by means of Student's t-test.

Values of p<0.05 are considered significant. LPS = lipopolysaccharide; PC = positive control (hydrocortisone); **p<0.01 vs LPS.

### 3. Study 1 results

The results obtained show that in the cells stimulated with LPS and treated with the mixture there isa significant reduction in both cytokines. In particular, TNF-α decreased significantly in the cells treated with the mixture at all the tested concentrations, while IL-6 decreased significantly in the cells treated with the mixture at concentrations of 0.02 and 0.01 mg/ml.

### Study 2: evaluation of the ability of the mixture to modulate cell proliferation in a prostate cell model 1. Materials and methods

### Cell cultures

The test was conducted on a normal human prostate cell line (WPMY-1), cultured in DMEM (Dulbecco's Modified Eagle Medium) containing 5% fetal bovine serum (FBS) and 1% antibiotics (penicillin and streptomycin) and incubated under standard culture conditions (37°C, 5% CO2).

### Sample and positive control preparation

The mixture tested in study 2 is: GREENSELECT^{®} PHYTOSOME 9 grams, red clover 7.50 grams, Teupol 10 P 1.50 grams, L-selenomethionine 0.33 grams, zinc citrate 1.44 grams.

The tested mixture was found to be soluble in dimethyl sulfoxide (DMSO) at a concentration of 2.0 mg/ml. It was then diluted in cell culture medium until reaching the final concentrations to be used in the test. Hesperidin (HES) was used as a positive control. It is known that HES improves the symptoms of BPH by attenuating cell proliferation, the inflammatory response and the epithelial-mesenchymal transition. HES was dissolved in DMSO at a concentration of 8.2 mM and then diluted in cell culture medium until reaching a final concentration of use of 50 µM.

### 2. Experimental protocol

### Preliminary cytotoxicity assays

A preliminary cytotoxicity assay was performed to identify the non-cytotoxic concentrations of the mixture.

WPMY-1 cells were seeded in 96-well plates at a density of 1x10⁴ cells/well, incubated overnight and then treated for 24 hours with different concentrations of the substances under examination. In order to prevent the solvent from interfering with the test result, the mixture dissolved in DMSO was diluted 100 times in culture medium before being administered to the cells.

Cell viability was assessed by means of an assay with MTT [3-(4,5-dimethylthiazol-2-yl)-2,5-diphenyltetrazolium bromide], a yellow-colored tetrazolium compound which is reduced by metabolically active cells into purple-colored formazan. The cells were treated with MTT (1 mg/ml) and incubated for 3 hours under standard conditions. At the end of this period, the MTT solution was eliminated and 100 µl of isopropanol were added in each well to dissolve the formazan crystals formed. The absorbance (optical density, OD) was determined by means of a spectrophotometer reading at a wavelength of 570 nm.

### Evaluation of PCNA expression

The WPMY-1 cells were seeded on coverslips, inserted into 6-well plates (1 coverslip/well) at a density of 1x10⁵ cells/well and incubated overnight under standard culture conditions. At the end of the incubation period, the seeded cells were pretreated and incubated for 60 minutes with 50 µM HES or with the tested mixture at three concentrations selected based on the results of the preliminary cytotoxicity test (0.02, 0.01 and 0.005 mg/ml). At the end of the pretreatment, DHT 320 nM (final) was added to promote induction, and the cells were incubated for 24 hours. A part of the seeded cells was not subjected to pretreatments or induction, whereas a part of the induced cells was not subject to treatments; the latter experimental conditions were tested to determine the baseline protein levels in the WPMY-1 cells and the effect of the induction protocol.

At the end of the period of contact, all the coverslips were fixed in methanol in order to then be labeled first with an anti-PCNA primary antibody, then a fluorophore-conjugated secondary antibody (Alexa Fluor 594) for the detection of fluorescence and lastly with a fluorescent nuclear dye (Hoechst 33528) to identify the nuclei and evaluate the protein localization.

For the purpose of acquiring images to be analyzed, the labeled coverslips were mounted on microscope slides with Mowiol left to polymerize overnight.

To enable a correct evaluation of the labeling process and rule out cell autofluorescence, the following internal checks were performed: fixing and simulation of the labeling process on non-induced/untreated cells, fixing and labeling only with the secondary antibody on non-induced/untreated cells.

Images were acquired with a Leica TCS SP8 confocal microscope at the Centro Grandi Strumenti of the University of Pavia and were analyzed with CellProfiler and ImageJ software.

### 3. Results

### Preliminary evaluations of cell viability

The mixture was dissolved in DMSO (2.0 mg/ml), diluted in cell culture medium and tested starting from a concentration of 0.02 mg/ml and with successive 1:2 dilutions (again in cell culture medium).

At the tested concentrations, no significant changes in cell viability are observed; therefore, in the subsequent test steps it is decided to use the concentrations of 0.02, 0.01 and 0.005 mg/ml (Table 7).

**Table 7: cell viability**

| | **NC** | **Mixture concentration (mg/ml)** | | | | | | | |
|---|---|---|---|---|---|---|---|---|---|
| | | 0.0002 | 0.0003 | 0.0006 | 0.0013 | 0.0025 | **0.005** | **0.01** | **0.02** |
| Mean OD | 1.005 | 1.080 | 1.076 | 1.025 | 1.009 | 1.059 | 1.048 | 1.052 | 0.936 |
| Viability (%) | 100.0 | 107.5 | 107.1 | 102.0 | 100.5 | 105.5 | 104.3 | 104.7 | 93.2 |

Absorbance measured at 570 nm is directly proportional to cell viability. The percentages shown in the table were calculated based on OD values at 570 nm and considering as 100% the OD of the negative control. NC = negative control (untreated cells).

### Evaluation of PCNA expression

The fluorescence intensity (FI) is directly proportional to the amount of PCNA present in the cells.

In order to assess PCNA expression in the induced cells, the percentages were calculated based on measured fluorescence values and represent the FI percentage compared to the negative control (cells not induced, NI).

| | | NI | | DHT 320 nM | | |
|---|---|---|---|---|---|---|
| Mean FI | | 11862.984 | | 9743.926 | | |
| % of NI | | 100.00 | | 82.14 | | |
| Change % | | - | | -17.86 | | |

**Table 8: PCNA expression levels in cells induced with DHT.**

| | NI | DHT 320 nM | | | | |
|---|---|---|---|---|---|---|
| | | + HES 50 µM | + TS 0.02 mg/ml | + TS 0.01 mg/ml | + TS 0.005 mg/ml | |
| Mean FI | 11862.984 | 2508.146 | 3066.064 | 9827.631 | 14141.757 | |
| % of NI | 100.00 | 21.14 | 25.85 | 82.84 | 119.21 | |
| Change % | - | -78.86 | -74.15 | -17.16 | 19.21 | |

| | | | | | | |
|---|---|---|---|---|---|---|
| HES = hesperidin; TS = Tested Sample, i.e. the tested mixture | | | | | | |

Figure 5 contains representative images with the highest fluorescence data for every experimental condition, acquired with a confocal microscope and processed with ImageJ software; they show the expression of the protein PCNA within the cell nuclei.

### 3. Study 2 results

The results obtained show a high baseline PCNA expression in cells not induced (NI) and no increase in PCNA expression was observed in cells induced with DHT. This indicates that the cells used in the test were in a phase of high proliferative activity, a condition already in itself attributable to a BPH intracellular environment.

Therefore, an evaluation of the effect of the tested mixture on the modulation of the protein's expression and the consequent statistical analysis were carried out by comparison with the NI cells.

The tested mixture significantly reduces PCNA expression. In particular, the effect is observed at concentrations of 0.02 mg/ml and 0.01 mg/ml, with a reduction of 74.15% and 17.16%, respectively.

The potential autofluorescence of the mixture and of the solvent used (DMSO) was also evaluated; the data obtained did not show levels of autofluorescence of the substances tested, thus ruling out their contribution to the reading of the fluorescent signal (data not shown).

## Claims

1. A mixture comprising or, alternatively, consisting of:
i) an epigallocatechin-3-gallate (EGCG) and/or an extract of *Camellia sinensis (L.) Kuntze*;
ii) one or more isoflavones selected in the group comprising or, alternatively, consisting of formononetin, daidzein, genistein, biochanin A and/or an extract of red clover *Trifolium pratense L. Leguminosa*; and
iii) a phenylpropanoid teupolioside and/or an extract of *Ajuga Reptans L.*

2. The mixture according to claim 1, wherein said mixture may further comprise:
iv) selenium or a salt thereof, preferably selected in the group comprising or, alternatively, consisting of L-selenomethionine, sodium selenite, sodium selenate, selenocysteine, more preferably L-selenomethionine; and/or
v) zinc or a salt thereof, preferably selected in the group comprising or, alternatively, consisting of zinc gluconate, zinc picolinate, zinc acetate, zinc bisglycinate, zinc sulfate and zinc citrate, more preferably zinc citrate.

3. The mixture according to claim 1 or 2, wherein said i) epigallocatechin-3-gallate (EGCG) may be present, in whole or in part, as a phytocomplex; more preferably, said phytocomplex may be present in a dry extract of *Camellia sinensis (L.) Kuntze*.

4. The mixture according to claim 3, wherein said dry extract of *Camellia sinensis (L.) Kuntze* may have a titer of epigallocatechin-3-gallate (EGCG) ≥ 13%; preferably, may have a titer comprised from 13% to 40%, preferably from 15% to 30%, more preferably from 19% to 25%, measured by HPLC.

5. The mixture according to any one of the preceding claims, wherein said i) epigallocatechin-3-gallate (EGCG) may be present in the form of a solid dispersion; preferably, in the form of a phytosomal solid dispersion.

6. The mixture according to any one of the preceding claims, wherein said ii) one or more isoflavones may be present, in whole or in part, as a phytocomplex; more preferably, said phytocomplex may be present in a dry extract of red clover *Trifolium pratense L. Leguminosa*.

7. The mixture according to claim 6, wherein said dry extract of *Trifolium pratense L. Leguminosa* has a titer of total isoflavones (formononetin, daidzein, genistein, biochanin A) ≥ 20%; preferably comprised from 20% to 50%, more preferably from 20% to 40%, even more preferably 20%, measured by HPLC.

8. The mixture according to any one of the preceding claims, wherein said iii) phenylpropanoid teupolioside may be present, in whole or in part, as a phytocomplex; more preferably said phytocomplex may be present in a dry extract of *Ajuga Reptans L.*

9. The mixture according to claim 8, wherein said dry extract of *Ajuga Reptans L.* may have a titer of phenylpropanoid teupolioside ≥ 10%, preferably comprised from 10% to 40%, more preferably comprised from 15% to 30%, even more preferably comprised from 20% to 25%, measured by HPLC.

10. The mixture according to any one of the preceding claims, wherein said mixture preferably comprises or, alternatively, consists of:
i) an extract of *Camellia sinensis (L.) Kuntze*;
ii) an extract of *Trifolium pratense L. Leguminosa*;
iii) an extract of *Ajuga Reptans L*.;
iv) selenium or a salt thereof, and/or selenium in an organic form such as L-selenomethionine; and
v) zinc or a salt thereof and/or zinc in an organic form such as zinc citrate.

11. The mixture according to claim 10, wherein said mixture preferably comprises or, alternatively, consists of:
i) an extract of *Camellia sinensis (L.) Kuntze* (titer ≥ 13%), preferably in the form of a phytosomal solid dispersion, in an amount by weight, relative to the total weight of the mixture, preferably comprised from 30% to 70%, preferably from 40% to 60%, more preferably from 45% to 55%;
ii) an extract of *Trifolium pratense L. Leguminosa* (titer ≥ 20%), comprising or, alternatively, consisting of formononetin, daidzein, genistein, biochanin A, in an amount by weight, relative to the total weight of the mixture, preferably comprised from 30% to 70%, preferably from 35% to 60%, more preferably from 40% to 50%;
iii) an extract of *Ajuga Reptans L.* (titer ≥ 10%), in an amount by weight, relative to the total weight of the mixture, preferably comprised from 2% to 20%, preferably from 5% to 15%, more preferably from 8% to 10%;
iv) selenium in an amount by weight, relative to the total weight of the mixture, preferably comprised from 0.007% to 0.02%, preferably from 0.008% to 0.01%, more preferably from 0.009% to 0.01%; and/or
v) zinc in an amount by weight, relative to the total weight of the mixture, preferably comprised from 0.5% to 10%, preferably from 1% to 5%, more preferably from 2% to 4%.

12. A composition comprising a mixture according to any one of claims 1-11 and, optionally, acceptable food or pharmaceutical grade excipients and/or additives.

13. The composition according to claim 12, comprising or, alternatively, consisting of:
- from 100 to 500 mg, preferably from 150 to 400 mg, even more preferably from 200 to 350 mg, even more preferably 300 mg of dry extract of *Camellia sinensis (L.) Kuntze* in the form of a phytosomal solid dispersion, preferably having a titer of epigallocatechin-3-gallate (EGCG) ≥ 13%, preferably comprised from 13% to 40%, preferably from 15% to 30%, more preferably from 19% to 25%, measured by HPLC;
- from 100 to 500 mg, preferably from 150 to 400 mg, even more preferably from 200 to 350 mg, even more preferably 250 mg of a dry extract of *Trifolium pratense L. Leguminosa*, preferably having a titer of total isoflavones (formononetin, daidzein, genistein, biochanin A) ≥ 20%, preferably comprised from 20% to 50%, more preferably from 20% to 40%, even more preferably 20%, measured by HPLC;
- from 1 to 200 mg, preferably from 10 to 100 mg, more preferably from 20 to 50 mg,even more preferably 50 mg of a dry extract of *Ajuga Reptans L.,* preferably having a titer of phenylpropanoid teupolioside ≥ 10%, preferably comprised from 10% to 40%, preferably from 15% to 30%, more preferably from 20% to 25%, measured by HPLC;
- from 45 to 65 µg, preferably from 50 to 60 µg, more preferably from 55 to 58 µg, even more preferably 55 µg of selenium or a salt thereof and/or selenium in an organic form;
- from 1 to 40 mg, preferably from 5 to 30 mg, more preferably from 10 to 20 mg, even more preferably 15 mg of zinc or a salt thereof and/or zinc in an organic form; and,
optionally, acceptable food or pharmaceutical grade excipients and/or additives.

14. The mixture according to any one of claims 1-11 or the composition according to one of claims 12-13, for use as a medicament.

15. The mixture or the composition according to claim 14, wherein said mixture and/or said composition are for use in a method, therapeutic or non-therapeutic, for reducing and/or treating prostate inflammation and symptoms associated therewith, and/or in a method for reducing and/or treating benign prostatic hyperplasia, more preferably for reducing and/or treating benign prostatic hyperplasia.

16. The mixture for use or the composition for use according to claim 15, wherein said inflammation is a chronic prostate inflammation (chronic prostatitis) and said symptoms associated with prostate inflammation are lower urinary tract symptoms (LUTS); wherein said lower urinary tract symptoms (LUTS) are preferably selected from the group comprising or, alternatively, consisting of: pollakiuria, nocturia, urinary urgency and incontinence, urinary hesitancy (difficulty/delay in urinating), reduced and intermittent urinary stream, burning during urination, and feeling of incomplete emptying.

17. The composition for use according to any one of claims 14-16, for oral use, preferably in the form of a capsule, a tablet, a sachet, a stick, an orodispersible stick, a gel for oral use or granules, more preferably in the form of a capsule; even more preferably in the form of a delayed-release capsule, said delayed-release capsule being based on HPMC and pectin.
